Europäisches Patentamt

European Patent Office

Office européen des brevets

(11) Publication number: 0 571 859 A2

# EUROPEAN PATENT APPLICATION

(21) Application number: 93108008.9

(51) Int. Cl.5: C07C 25/18, C07C 17/02

(22) Date of filing: 17.05.93

(30) Priority: 26.05.92 US 887909
17.08.92 US 930809

(43) Date of publication of application:
01.12.93 Bulletin 93/48

(84) Designated Contracting States:
DE FR GB NL

(71) Applicant: ETHYL CORPORATION
451 Florida Boulevard
Baton Rouge, Louisiana 70801(US)

(72) Inventor: Hussain, Saadat
5321 Highland Ridge Drive
Baton Rouge, Louisiana 70817(US)
Inventor: McKinnie, Bonnie Gary
261 Columbia 10W
Magnolia, Arkansas 71753(US)
Inventor: Ransford, George Henry
2500 Fox Run
Magnolia, Arkansas 71753(US)
Inventor: Parks, John C.
18444 Weatherwood Drive
Baton Rouge, Louisiana 70817(US)
Inventor: DeVrou, Phillip R.
721 North Washington
Magnolia, Arkansas 71753(US)

(74) Representative: Sandmair, Kurt, Dr. Dr.
Patentanwälte
Schwabe, Sandmair, Marx
Stuntzstrasse 16
D-81677 München (DE)

(54) A decabromodiphenylethane predominated product having enhanced whiteness.

(57) A decabromodiphenylethane-predominant product having a Y.I. in the range of 5 to <11 is obtained by reacting diphenylethane with bromine at a rate such as to minimize color-causing side reactions. In a preferred embodiment of the invention, the diphenylethane is fed below the liquid level of bromine in a reaction vessel through a narrow-diameter dip tube at a rate of 50-800, most preferably 250-600 cm/second.

This invention relates to a decabromodiphenylethane-predominant product having a yellowness index <11.

Decabromodiphenylethane is a known flame retardant which is expected to increase in commercial significance because of its not being associated with the production of dioxin and other undesirable compounds under thermoplastic blending or molding conditions. However, when prepared in the form in which it will most probably be provided in commercial quantities (i.e., as an impure product containing at least 90 wt % decabromodiphenylethane), it has too much color to make it desirable for use in flame retarding articles which are intended to have a white or other light color. The undesirable color of the impure product is believed to be at least partially due to its morphology and/or to the presence of chromophoric impurities - both of which can be attributed to the particular impurities in the reactants and to the peculiar characteristics of the process used in preparing the product.

Although decabromodiphenylethane-predominant products having ASTM D 1925 Yellowness Index (hereinafter Y.I.) values in the range of about 11-15 have been prepared, such products are not close enough to the ideal of the pure white product having a Y.I. of 0.0. What is still needed is a Y.I. value <11, preferably a Y.I. value ≤7, since Y.I. values ≤7 appear very near to the ideal of pure white to the human eye.

It has now been found that reacting diphenylethane with bromine at a rate such as to minimize color-causing side reactions permits the formation of a decabromodiphenylethane-predominant product (i.e., an impure product containing at least about 90 wt %, preferably 95-99.5 wt %, and most preferably 98-99.5 wt % decabromodiphenylethane) having a Y.I. in the range of 5 to <11, preferably 5-8.5, and most preferably 5-7.

The major ingredient of a product of this invention, i.e., decabromodiphenylethane, can be any polybromodiphenylethane wherein the average number of ar-bromo substituents per molecule is ≤10 but >9 in accordance with the simplified system adopted in the flame retardant art for naming the mixtures which typically result from the bromination of aromatic compounds, such as diphenyl oxide and diphenyl ethane.[1]

Typically, the distribution for the bromo-homolog mixture composing the decabromodiphenylethane portion of a product of the invention will include, in gas chromatography (GC) area percentages, 0-1.5% octabromodiphenylethane and 0-8% nonabromodiphenylethane, with the balance - preferably at least 95% - being decabromodiphenylethane. A preferred distribution is 1-2% nonabromodiphenylethane and 98-99% decabromodiphenylethane.

The impurities in the products of the invention can include one or more of bromine, nonabromodiphenylethane, dodecabromodiphenylethane, aluminum salts, sodium bromide, water, brominated olefins (e.g., brominated stilbene), and brominated diphenylmethane - the bromine usually being the most predominant impurity from a quantitative view. Depending on the amount of free bromine present in the crude dry product, the dodecabromodiphenylethane content can be 0-10,000 ppm, while the other impurities are generally present in amounts of 0-500 ppm. Morphologically, the decabromodiphenylethane constituent is believed to have some crystallinity - the major morphological quality being amorphous.

Strictly speaking, the decabromodiphenylethane-predominant products of the invention do not have a melting point (m.p.), since they melt over a range of temperatures rather than at a single temperature. However, since they typically begin to melt at a temperature of 344-346° C and complete melting at a temperature of 346-354° C, it is convenient to describe them as having a m.p. range (or m.p.) of 344-354° C. Actually, the products exhibit rather narrow m.p.'s, e.g., 346-350° C or 348-354° C in preferred embodiments.

The products of this invention contain ≤500 ppm, preferably ≤100 ppm free bromine, i.e., bromine which is present in the form of $Br_2$. Another of their more desirable qualities is the good thermal stability evidenced by their experiencing <15% weight loss at 400° C when subjected to thermogravimetric analysis. A preferred thermogravimetric weight loss profile is as follows:

[1] As is known, such brominations result in the formation of mixtures of homologs, with one or two of the homologs comprising the greatest percentage of all of the homologs present; and the simplified nomenclature system is to name it as the homolog containing the average number of ar-bromo substituents per molecule or, when that average is a mixed number, the homolog containing the next whole number - a polybromodiphenyl oxide containing an average of 7.3, 7.8, or 8.0 ar-bromo substituents per molecule, e.g., thus being referred to as octabromodiphenyl oxide.

| Temperature | % Weight Loss |
|---|---|
| 200 °C | <0.1 |
| 300 °C | <0.5 |
| 400 °C | <10.0 |

The process which is modified to permit the formation of the present decabromodiphenylethane-predominant products is one in which the reaction between diphenylethane and bromine is conducted by feeding liquid diphenylethane to a reaction vessel containing liquid bromine and a bromination catalyst so that the diphenylethane is introduced below the liquid level of the bromine. In this process, the amount of diphenylethane employed is ordinarily 0.03-0.06, preferably 0.035-0.055, and most preferably 0.037-0.043 mol/mol of bromine; the reaction temperature is in the range of 15° C to reflux, preferably 30-80° C, and more preferably 50-60° C; and it is preferred that the product have an average particle size large enough to permit its being easily recovered by filtration.

In the practice of the present invention, this process is modified so that the diphenylethane is contacted with the bromine and bromination catalyst at a rate such as to minimize color-causing side reactions. It has been discovered that increasing the rate at which each molecule of diphenylethane reacts with ten molecules of $Br_2$ can provide products having improved color characteristics, as well as desirable product size. Thus, on a microscopic basis, each mole of diphenylethane in a droplet thereof should be mixed with at least ten moles of $Br_2$ in the shortest time possible. This "micro-mixing" of the reactants can be achieved in a number of ways, such as by increasing the agitation rate during the diphenylethane feed or by decreasing the dip tube diameter while increasing the diphenylethane feed rate - the latter generally being preferred because of its being less difficult and costly than increasing the agitation rate on a commercial scale.

What is believed to happen in the practice of the invention is that the high velocity feed rate achieved with a small diameter dip tube provides a jet stream of diphenylethane which entrains sufficient bromine on a microscopic scale to perbrominate adjacent diphenylethane molecules in the jet stream - the time required to entrain sufficient bromine being decreased (i.e., the micro-mixing being increased) as the diameter of the dip tube is decreased and the feed rate is increased. This theory is supported by time-temperature measurements made during diphenylethane/bromine reactions in which the diphenylethane is fed (A) at a rate of 447 cm/second through a dip tube having an inner diameter of 4.76 mm, (B) at a rate of 63 cm/second through a dip tube having an inner diameter of 12.7 mm, or (C) at a rate of 15.7 cm/second through a dip tube having an inner diameter of 24.4 mm. These measurements show that the peak temperature (the temperature at which a microscopic droplet of diphenylethane has theoretically mixed and reacted with 10 mols $Br_2$/mol diphenylethane) in these reactions occurs at (A) ~0.02 second, (B) ~0.3 second, and (C) ~2.1 seconds.

The same effect of maximizing reaction rate by increasing the micro-mixing can also be achieved by increasing the agitation rate in the reaction vessel, although the effect of agitation is more noticeable in small-scale reactions than on a commercial scale.

Regardless of the particular manner in which the reaction rate is maximized, however, it decreases the formation of color-forming impurities; and it has the added advantage of providing the larger particle size (i.e., preferably >10$\mu$, more preferably > 12$\mu$, and most preferably >15$\mu$) desirable to facilitate filtration.

It is preferred that the bromine charged to the reactor be essentially anhydrous (i.e., <50 ppm water) and contain little, if any, iron and ≤10 ppm, preferably <5 ppm, organic impurities, e.g., oil, grease, and carbonyl-containing hydrocarbons, so that there will be little, if any, impact on the color attributes of the product. When commercial-grade bromine having such a purity is not available, the organic impurity and water contents of the bromine can be conveniently reduced by mixing bromine and concentrated (94-98%) sulfuric acid in a 3/1 volume ratio; stirring the resultant two-phase mix for 10-16 hours; then separating the sulfuric acid phase, along with the impurities and water, from the bromine phase; and, if desired, subjecting the recovered bromine phase to distillation to enhance its purity.

The bromination catalyst used in the process is preferably $AlCl_3$ and/or $AlBr_3$, although use may be made of other bromination catalysts having sufficient catalytic activity under the reaction conditions, such as aluminum powder, iron powder, $FeCl_3$, $FeBr_3$, $ZrCl_4$, or $ZrBr_4$ alone or in combination with the aluminum trihalide(s). These catalysts are used in catalytic quantities, typically 0.1-20 wt %, preferably 6-15 wt %, and most preferably 8-11 wt %, based on the weight of the diphenylethane - the amount preferably being kept as low as is possible without deleteriously affecting perbromination. Based on the weight of bromine, a catalyst concentration of 0.38-0.45 wt % is especially useful.

The bromination catalyst and bromine can be charged to the reaction vessel in any order or together. Care should be taken not to aspirate atmospheric moisture into the reaction vessel, as the presence of moisture can partially or totally deactivate the catalyst.

The amount of bromine charged to the reaction vessel should be sufficient to effect the desired degree of bromination and to provide an easily stirred reaction mass. This amount is generally a stoichiometric excess, preferably a stoichiomeric excess > 125%, most preferably 150-175%. After completion of the reaction, the unreacted bromine will be a liquid component of the reaction mass and will continue to serve the aforementioned function of facilitating maintenance of a stirrable reaction mass.

The diphenylethane employed may be a commercial material, or it may be synthesized by known techniques, e.g., by the reaction of benzene and ethylene dihalide or by the oxidative dimerization of toluene at a temperature of ≧400°C in the presence of a metal oxide catalyst to form diphenylethane and diphenylalkene, followed by hydrogenation to remove the olefinic unsaturation in the by-product.

It is not uncommon for the diphenylethane reactant to contain various impurities, such as diphenylmethane, stilbene, benzene, alkylated diphenylethane and diphenylmethane, and tetrahydronaphthalene, which may be detrimental to the color of the final product. When these impurities become troublesome, their amounts can be reduced by conventional means, e.g., purification of the diphenylethane by recrystallization.

The diphenylethane is fed to the reaction vessel in the molten state at a temperature which is above its melting point (53-55°C) but not high enough to cause diphenylethane degradation - preferably a temperature in the 55-80°C range. Since the lower viscosity of the molten diphenylethane at the higher temperatures makes it easier to feed, the most preferred temperatures are 70-80°C.

It is preferred that the molten diphenylethane be blanketed by a non-oxidizing atmosphere until it is fed into the reaction vessel to prevent or minimize the production of oxidative decomposition impurities (e.g., 1-hydroxy-1,2-diphenylethane, benzaldehyde, and benzyl alcohols) that could adversely affect the color of the final product. Such an atmosphere can be provided by most inert gases, e.g., nitrogen, argon, neon, helium, krypton, and xenon.

The addition of the diphenylethane below the liquid level of the bromine in the reaction vessel is an important process feature which permits a reaction product having a high average bromine number to be obtained more quickly than when the liquid diphenylethane is fed above the liquid surface of the bromine. The depth below the liquid bromine surface at which the feed is to occur is that which is sufficient to obviate or diminish splattering of the reaction mass during the feed. Generally, a depth such as 0.5-1.0 inch (1.3-2.5 cm) in laboratory-scale equipment and 0.5-6.0 feet (0.15-1.83m) in commercial-scale equipment is suitable, and a depth of about 0.5 inch (1.3 cm) will be functional in almost all cases.

Whether the reaction is conducted in small-scale or commercial-scale equipment, the diphenylethane feed rate should be selected so as to be consistent with the need to control the reaction temperature and HBr evolution, just as the degree of agitation should be chosen so as to achieve the fast reaction rate while keeping the fracturing of product particles to a minimum. In some cases it may be desirable to use multiple dip tubes to keep the overall diphenylethane feed rate at a level which gives good process efficiency.

In a commercial process, sufficient micro-mixing is provided by the use of agitation rates of 20-150 revolutions per minute (rpm) and dip tubes having inner diameters of 0.48-1.6 cm, especially 0.48 cm or 1.3 cm; and the diphenylethane feed rates are preferably in the range of 50-800, more preferably 200-700, and most preferably 250-600 cm/second. When the reaction is conducted on a small scale, agitation rates of 90-130 rpm and dip tubes having inner diameters of 0.5-1.25 mm are suitable - a preferred dip tube inner diameter being 0.75 mm.

Since the bromination of diphenylethane is exothermic, cooling of the reaction mass during the diphenylethane feed may be needed to maintain the desired temperature. The heat of reaction can be removed by cooling the reaction vessel or by having the reaction mass under reflux conditions so that heat can be removed by the use of an overhead condenser. During the diphenylethane feed, the temperature of the reaction mass is preferably at least 45°C and most preferably above the melting point of the diphenylethane. Lower temperatures can be used, but they require a diphenylethane feed velocity sufficiently high to prevent freeze-up of the molten feed in the dip tube which is in contact with the relatively cool reaction mass.

The process can be run so that the pressure in the reaction vessel provides a refluxing condition at the selected reaction temperature. With a refluxing condition, control of the reaction temperature is facilitated. If temperature control is effected otherwise, e.g., by the use of heating or cooling jackets, the pressure can be any which is not prohibitive of the obtainment of the various defined parameters of the process. Also, since temperatures above the boiling point of bromine are useful in the process, superatmospheric pressures, e.g., 5 psig (34.5 kPa), can be desirable.

After the diphenylethane feed is partially complete, the overhead can be blocked to allow the reaction vessel pressure to build as more and more HBr is produced. Once the diphenylethane feed is completed, the reaction mass is then discharged from the reaction vessel by the simple expedient of using the built-up pressure in the reaction vessel. Generally, no significant post-feed cook period is required, and the discharge can occur as soon as is practical after the bromination reaction ceases.

Since the bromination reaction is a substitution reaction, HBr will be evolved as long as bromination is occurring. Hence, monitoring of the reaction for the cessation of HBr evolution can be used as an indicator to determine when substantially perbrominated reaction product has been obtained.

After the reaction has at least substantially ceased, the reaction mass comprises a mixture of a liquid (which comprises mostly bromine) and a solid which comprises brominated diphenylethane, entrained bromine, and other impurities. The recovery of the brominated diphenylethane product and its entrained bromine can be effected conventionally, e.g., by steam stripping or filtration - steam stripping being frequently preferred because of its not only separating the liquid, i.e., the remaining non-entrained bromine, from the solids but its also deactivating the catalyst. An advantage of the products of this invention is that the solid product does not tend to aggregate during the steam stripping step.

After the steam strip, the remaining solids, which are predominant in decabromodiphenylethane, can be first washed with an aqueous base (e.g., aqueous NaOH or $Na_2CO_3$) to neutralize and remove HBr and free $Br_2$; and the product is then water-washed. The washed product is further treated to remove entrained bromine, which is deleterious to color and to product quality. This removal can be effected by (1) oven-aging the product at a temperature in the 200-250° C range for 6-20 hours and then fracturing the product, e.g., by grinding or milling, or (2) fracturing the product and then oven-aging. Interestingly, better color is achieved by the latter procedure.

The decabromodiphenylethane-predominant product of this invention may be used as a flame retardant in formulations containing virtually any flammable material, including cellulosic materials and polymers. Illustrative of the polymers are homopolymers of olefins, such as ethylene, propylene, and butylene; interpolymers of such olefins with one another and/or with other copolymerizable monomers, e.g., ethylene/propylene, ethylene/ethyl acrylate, and ethylene/vinyl acetate copolymers; polymers of other olefinically-unsaturated monomers, e.g., polystyrene, styrene copolymers, and high impact polystyrenes; polyurethanes; polyamides; polyimides; polycarbonates; polyethers; acrylic resins; phenolics; alkyds; polyesters, such as poly(ethyleneterephthalate) and poly(butyleneterephthalate);epoxy resins; elastomers, such as butadiene/styrene and butadiene/acrylonitrile copolymers; acrylonitrile/butadiene/styrene terpolymers; butyl rubber; natural rubber; and polysiloxanes. Such polymers may be crosslinked by chemical means or by irradiation, if desired, or may be uncrosslinked; and they be single polymers or blends thereof.

The amount of product used in a formulation will be that needed to obtain the desired flame retardancy. As will be apparent to the practitioner, this amount will vary with the particular flammable material, the presence of other additives, the degree of flame retardancy sought, and the shape of the article into which the formulation is to be made - electrical insulation, tubing, and film, e.g., each behaving differently. In general, however, the formulation may contain 5-40, preferably 10-30 wt % of the product when it is the only flame retardant compound in the formulation.

The quantity of product needed to achieve a given flame-retardancy is reduced when it is used together with an inorganic compound, especially $Fe_2O_3$, ZnO, zinc borate, or the oxide of a Group V element, e.g., Bi, As, P, or Sb - $Sb_2O_3$ being particularly preferred. When thus used, the product/inorganic compound weight ratio in the system is generally 1-7/1, preferably 2-4/1; and the system may constitute up to 40%, preferably 20-30% of the weight of the formulation.

Formulations containing the products of the invention may also contain any of the additives usually present in such formulations, e.g., plasticizers, antioxidants, fillers, pigments, and UV stabilizers; and articles formed from formulations containing a thermoplastic polymer and a product of the invention may be produced conventionally, e.g., by injection, extrusion, or compression molding.

The following examples are given to illustrate the invention and are not intended as a limitation thereof.

EXAMPLE 1

Part A

Charge a suitable 500 mL reaction vessel with 258 mL bromine and 3.6g $AlCl_3$ and then with molten diphenylethane obtained by grinding 36.4g (0.2 mol) of 99.9% pure diphenylethane having a water content of 356 ppm and charging it to an addition funnel wherein it is heated slowly with a heat gun. Add the molten diphenylethane over a period of 92 minutes through a dip tube having a nominal inner diameter of 0.75 mm

5

and positioned so that its end is 0.75 inch (1.9 cm) below the liquid bromine level in the vessel - agitating the reaction mixture with a stirrer run at 150 rpm and maintaining the molten diphenylethane at 55-66° C and the vessel temperature at 55° C during the addition. After completing the addition, reflux the reaction mixture at 60° C for 0.7 hour, allow it to cool to 40° C over a 30-minute period, add 75 mL of water, and steam distill the mixture until a vaporhead temperature of 97° C is obtained. Add 60 mL of water and 60 mL of a 25% aqueous NaOH to the remaining mass, centrifuge the resultant slurry, wash the recovered solids with deionized water until neutral, and oven-dry the washed solids at 100° C for one hour. Then jet-mill the dried product to obtain an average particle size of 2.5-3.5$\mu$ and oven-age the milled product for six hours at 230° C.

Part B

Repeat Part A except for oven-aging the dried product before dry milling it.

EXAMPLE 2

Repeat Example 1, Part A, except for feeding the diphenylethane over 77 minutes.

EXAMPLE 3

Repeat Example 1, Parts A and B, except for feeding the diphenylethane over 95 minutes.

COMPARATIVE EXAMPLE A

Repeat Example 1, Part B, except for (1) using a dip tube having a nominal inner diameter of 4.75 mm and a bromine charge which has been pretreated by being stirred over $H_2SO_4$ for six hours, (2) adding the diphenylethane over 94 minutes, (3) refluxing the reaction mixture for one hour, (4) adding 300 mL of water to the reaction mass, (5) ceasing steam distillation when the overhead temperature reaches 100° C, (6) treating the reaction mass remaining after steam distillation with 25 mL of a 50% aqueous NaOH, and (7) air-drying the water-washed solids overnight.

COMPARATIVE EXAMPLE B

Repeat Comparative Example A except for drying the diphenylethane under nitrogen at 75-80° C for four hours before feeding it.

EXAMPLE 4

Determine the color of the products of the preceding examples in accordance with ASTM D 1925 and with a Hunter Lab Model Colorquest 45°/0°, calculating the Y.I. values by the equation Y.I. = 100(0.72a + 1.79b)/L, wherein "a" is an observed value which measures redness when a plus value, gray when zero, and greeness when a negative value; "b" is an observed value which measures yellowness when a plus value, gray when zero, and blueness when a minus value; and "L" is an observed value which measures lightness and varies from 100, for perfect white, to 0, for black - L values of 90-95 being those preferred for products of the invention.

The calorimetric values determined for the products are shown in Table I.

TABLE I

| Example | Dip Tube Inside Diameter | Post-treatment | Hunter Values a, b, L | Y.I. |
|---|---|---|---|---|
| 1-A | 0.75 mm | Jet-milled then Oven-aged | a = -0.16<br>b = +4.11<br>L = 93.5 | 7.7 |
| 1-B | 0.75 mm | Oven-aged then Jet-milled | a = -0.10<br>b = +3.55<br>L = 90.6 | 6.9 |
| 2 | 0.75 mm | Jet-milled then Oven-aged | a = -0.19<br>b = +3.68<br>L = 94.2 | 6.8 |
| 3-A | 0.75 mm | Jet-milled then Oven-aged | a = -0.23<br>b = +4.39<br>L = 94.2 | 8.1 |
| 3-B | 0.75 mm | Oven-aged then Jet-milled | a = -0.24<br>b = +3.73<br>L = 89.9 | 7.2 |
| A | 4.75 mm | Oven-aged then Jet-milled | a = -0.26<br>b = +6.11<br>L = 87.8 | 12.3 |
| B | 4.75 mm | Oven-aged then Jet-milled | a = -0.30<br>b = +5.62<br>L = 88.2 | 11.1 |

EXAMPLE 5

Using the same general procedure as in the previous examples, conduct a series of runs by feeding 571-1239 kg of molten diphenylethane to a 15,000-liter reaction vessel containing a 130-230% stoichiometric excess of bromine and 0.3-0.5% catalyst, based on the weight of diphenylethane. Conduct the reaction at 57°C in each run, and heat-treat each of the products at 230°C for six hours after drying and grinding the particles to an average of $7\mu$. The nominal inner diameters of the dip tubes, the diphenylethane feed rates, and the agitator speeds employed, as well as the pre-grinding particle size of the product and the Y.I. of the final product are shown in Table II.

TABLE II

| Run | 5-A | 5-B | 5-C | 5-D |
|---|---|---|---|---|
| Dip tube inner diameter | 4.76 | 4.76 | 12.7 | 12.7 |
| Feed rate (cm/sec) | 422 | 335 | 64 | 61 |
| Agitator RPM | 50 | 60 | 80 | 50 |
| Particle size ($\mu$) | 26.2 | 22.4 | 27.2 | 34.1 |
| Y.I. | 10.9 | 10.9 | 10.7 | 10.7 |

COMPARATIVE EXAMPLE C

Repeat Example 5, Run A, except for using a diphenylethane feed rate of only 280 cm/second. The Y.I. of the product is 13.5.

COMPARATIVE EXAMPLE D

Conduct two runs (D-1 and D-2) by essentially repeating Example 5, Run C, except for using a dip tube having a nominal inner diameter of 25.4 mm and feeding the diphenylethane at rates of 7.4 and 8.8 cm/second, respectively. The Y.I. values of the products are, respectively, 15.1 and 11.8. When Comparative Example D is essentially repeated except for using an agitator speed of 100 rpm and a diphenylethane feed rate of 6.6 cm/second, the Y.I. of the product is 12.3.

As demonstrated in the preceding examples, high Y.I. values are obtained when the diphenylethane is fed at low velocities, such as the 5-10 cm/second achieved with dip tubes having relatively large inner diameters. However, feeding the diphenylethane at a greater velocity permits lowering the Y.I. value of a product by increasing the diphenylethane feed rate and/or the agitation speed.

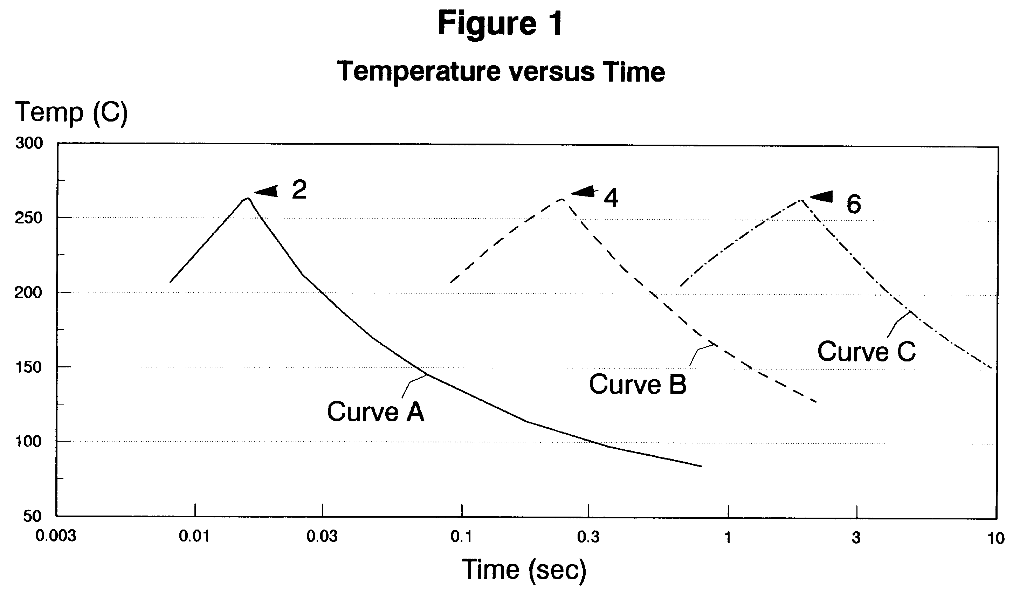

**Claims**

1. A decabromodiphenylethane-predominant product having a Y.I. value in the range of 5 to <11, as measured by ASTM D 1925.

2. The product of claim 1 having an "L" value of 90-95, as measured by ASTM D 1925.

3. The product of claim 1 wherein the Y.I. value is 5-8.5.

4. The product of claim 3 wherein the Y.I. value is 5-7.

5. A process for preparing a decabromodiphenylethane product by feeding diphenylethane at a level below the liquid level of the bromine to a reaction vessel containing bromine and a bromination catalyst and reacting the diphenylethane and bromine at a temperature in the range of 15° C to reflux; characterized in that the diphenylethane is contacted with the bromine and bromination catalyst at a rate sufficient to minimize side reactions.

6. The process of claim 5 wherein the rate at which the diphenylethane is fed into the reaction vessel is 50-800 cm/second.

7. The process of claim 6 wherein the rate is 250-600 cm/second.

8. The process of claim 5 wherein a >125% stoichiometric excess of bromine is reacted with the diphenylethane at 30-80° C.

9. The process of any of claims 5-8 wherein the decabromodiphenylethane product is recovered as a product having an average particle size >15$\mu$ and subsequently heat-treated to provide a decabromodiphenylethane-predominant product having a Y.I. value in the range of 5 to <11, as measured by ASTM D 1925.

10. The process of claim 5 wherein a > 125% stoichiometric excess of bromine is reacted with the diphenylethane at 30-80° C, the rate at which the diphenylethane is fed into the reaction vessel is 250-600 cm/second, and the decabromodiphenylethane product is recovered as a product having an average particle size >15$\mu$ and subsequently heat-treated to provide a decabromodiphenylethane-predominant product having a Y.I. value in the range of 5 to <11, as measured by ASTM D 1925.

# Figure 1

## Temperature versus Time

Temp (C)

EP 0 571 859 A2